# EUROPÄISCHE PATENTSCHRIFT

(11) **EP 1 290 977 B1**
(45) Veröffentlichungstag und Bekanntmachung des Hinweises auf die Patenterteilung: **27.10.2004**
(21) Anmeldenummer: 02019275.3
(22) Anmeldetag: 28.08.2002
(51) Int. Cl.: A61B 5/15

(54) **Lanzette für die Blutentnahme**
Lancet for sampling blood
Lancette de prélèvement du sang

(30) Priorität: 05.09.2001 DE 20114658 U
(43) Veröffentlichungstag der Anmeldung: 12.03.2003
(73) Patentinhaber: Wilden AG, 93051 Regensburg (DE)
(72) Erfinder: Argauer, Herbert, 92712 Pirk (DE)
(74) Vertreter: Schmidt, Horst, Dr.

(56) Entgegenhaltungen:
- EP-A- 0 589 186
- WO-A-96/02189
- GB-A- 2 352 403
- US-A- 3 358 689

## Beschreibung

Die Erfindung betrifft eine Lanzette für die Blutentnahme und insbesondere eine solche mit einer in einem Kunststoff-Basisteil gehaltenen Lanzettennadel aus einem metallischen Material, von der ein angespitzter Endbereich aus dem Basisteil herausragt, der in einem am Basisteil über eine Sollreissstelle angeformten Kunststoff-Kopfteil eingebettet ist, der vor Gebrauch unter Freilegen des Nadelendbereiches vom Basisteil entfernt werden kann, wenn auf einen der Teile eine Drehkraft relativ zum anderen Teil ausgeübt wird.

Derartige Lanzetten sind z.B. aus der EP-A-589186 bekannt und in ähnlicher Ausführung seit langem im praktischen Einsatz. Der Kopfteil, in dem der aus dem Basisteil herausragende zugespitzte Endbereich der Lanzettennadel eingebettet ist, stellt sicher, dass bis zur Verwendung der Lanzette der Nadelendbereich in einer sterilen Umgebung gehalten ist. Die bisherige Praxis bei der Handhabung solcher Lanzetten besteht darin, dass vor Gebrauch der Lanzette der Kopfteil vom Basisteil abgetrennt wird, indem man den Kopfteil um ein gewisses Mass gegenüber dem Basisteil verdreht, so dass die Sollreissstelle durchtrennt wird. Danach wird auf den Kopfteil von Hand eine Ziehkraft in mehr oder weniger axialer Ausrichtung zur Lanzettennadel ausgeübt, um die Reibkraft zwischen dem Nadelendbereich und dem Kopfteil zu überwinden, so dass der Kopfteil von dem Nadelendbereich abgezogen werden kann. Die Praxis hat jedoch gezeigt, dass häufig der Endbereich der Lanzettennadel so fest im Kopfteil sitzt, dass gelegentlich die Lanzettennadel bei dem geschilderten Vorgang aus dem Basisteil herausgezogen wird oder die aufzubringenden Ziehkräfte so gross sind, dass, wenn sie nicht genau in der Axial- oder Längsrichtung der Lanzettennadel wirken, es zu einem Verbiegen des Nadelendbereiches kommen kann. Lanzetten der in Rede stehende Art werden im allgemeinen in Verbindung mit Betätigungseinrichtungen verwendet, die eine schnelle mechanisierte Bewegung des Nadelendbereiches in die Haut einer Person zum Zwecke der Blutentnahme ermöglichen. Es wurde festgestellt, dass beim Abtrennen des Kopfteils häufig der in der Betätigungseinrichtung klemmend gehaltene Basisteil der Lanzette aus der Betätigungseinrichtung herausgezogen und die Lanzette damit unbrauchbar wurde.

Der Erfindung liegt die Aufgabe zugrunde, eine Lanzette der gattungsgemässen Art zu schaffen, die eine Trennung des Kopfteiles vom Basisteil ermöglicht, ohne dass dabei unerwünschte oder schädliche Auswirkungen auf die Lanzettennadel oder Probleme bei der Handhabung der Lanzette in Betätigungseinrichtungen befürchtet werden müssen.

Diese Aufgabe wird erfindungsgemäss durch die Merkmale des Anspruchs 1 gelöst. Insbesondere ist bei einer Lanzette mit dem eingangs erwähnten Aufbau eine zwischen dem Kopfteil und dem Basisteil angeordnete Nockenführungseinrichtung vorgesehen ist, die bei einer Relativdrehung zwischen dem Kopfteil und dem Basisteil eine axiale Bewegung des Kopfteiles relativ zum Basisteil bewirkt. Statt einer mehr oder weniger unkontrollierten Handhabung zum Abziehen des Kopfteils vom Nadelendbereich gewährleistet die Nockenführungseinrichtung eine gesteuerte axiale Bewegung des Kopfteils weg vom Basisteil unter weitgehender Entlastung der Lanzettennadel von axialen Kräften vor und während des Abtrennens des Kopfteils, da der Kopfteil bei diesem Vorgang weiterhin am Basisteil abgestützt ist. Ein Herausziehen der Lanzettennadel aus dem Basisteil oder des Basisteils aus einer Betätigungseinrichtung braucht deshalb nicht befürchtet zu werden. Ausserdem übernimmt die Nockenführungseinrichtung gleichzeitig die Funktion eines Knickschutzes, der ein Verbiegen des Nadelendbereiches unter seitlich auf den Kopfteil einwirkenden äusseren Kräften zuverlässig verhindert. Man hat zwar schon vorgeschlagen, am Kopfteil einen Knickschutz vorzusehen, der verhindern soll, dass eine seitliche Auslenkung des Kopfteiles über ein gewisses Mass hinaus nicht möglich ist, jedoch konnten die erwähnten Handbanbungsprobleme der Lanzette nicht behoben werden. Das vollständige Abtrennen des Kopfteils vom Basisteil erfordert, anders als bei den bekannten Lanzetten, nur eine kurze Drehbewegung des Kopfteils, ohne dass der Anwender hierbei umgreifen muss. Die Nockenführungseinrichtung kann gemäss einer Weiterbildung der Erfindung wenigstens ein Paar versetzt zueinander angeordnete Nockenbereiche am Kopfteil und wenigstens ein Paar versetzt zueinander und zu den Nockenbereichen am Kopftteil angeordnete Nockenbereiche am Basisteil umfassen, die mit den Nockenbereichen am Kopfteil bei der Drehung in Eingriff treten, wobei wenigstens die Nockenbereiche eines Paares Nockenführungsflächen aufweisen. Die Nockenbereiche lassen sich problemlos zusammen mit den übrigen Aufbaumerkmalen im Zuge der bevorzugten einstückigen Ausbildung der Lanzette im Kunststoff-Spritzgiessverfahren vorsehen.

Die Erfindung wird nachfolgend anhand der Zeichnung und einer Ausführungsform näher erläutert. Es zeigen:

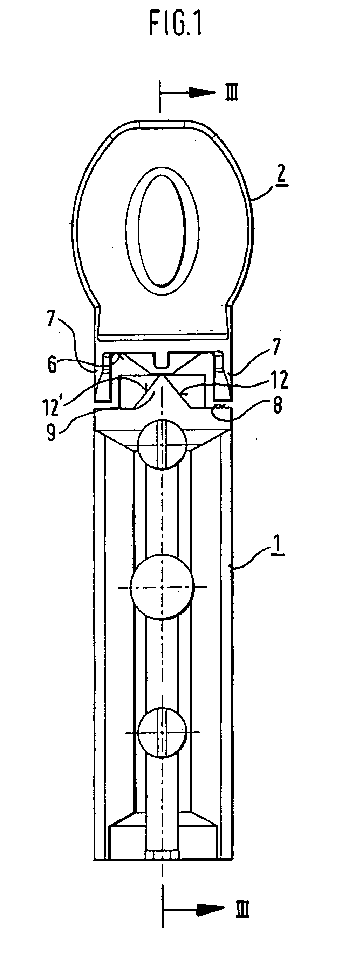

- Fig. 1: in einer Ansicht von einer Seite eine Lanzette gemäss einer Ausführungsform der Erfindung,

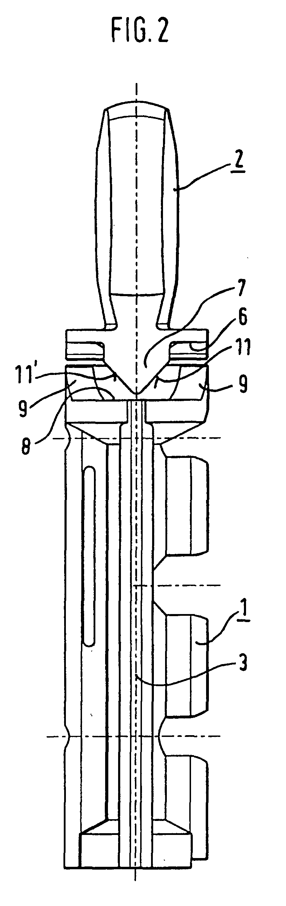

- Fig. 2: in einer Ansicht ähnlich Fig. 1 die Lanzette nach Drehung um 90°, und
- Fig. 3: in einer längsgeschnittenen Ansicht die Lanzette längs der Schnittlinie III-III in Fig. 1.

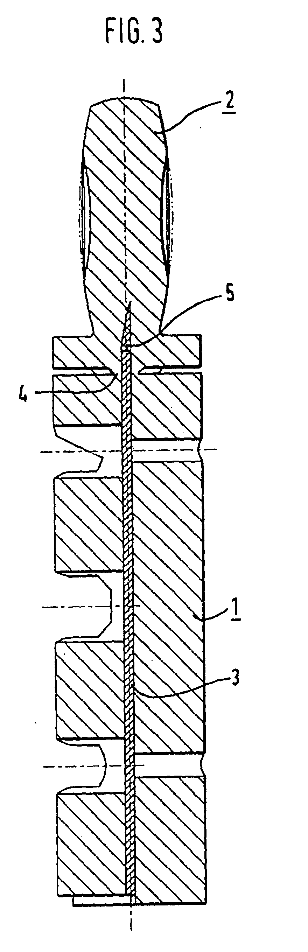

Die Lanzette gemäss der Erfindung umfasst einen Basisteil 1 und einen Kopfteil 2 sowie eine im Basisteil 1 gehaltene Lanzettennadel 3. Der Basisteil 1 und der Kopfteil 2 sind über eine Sollreiss- oder -bruchstelle 4, vgl. Fig. 3, integral miteinander verbunden. Die Lanzette stellt vorzugsweise eine Spritzgussteil aus einem geeigneten Kunststoffmaterial wie einem Polyolefin, z.B. Polypropylen oder Polyethylen dar, doch können auch andere Kunststoffmaterialien, wenn erwünscht, verwendet werden. Die Sollbruchstelle 4 ist so ausgelegt, dass sie unter der Einwirkung einer äusseren Kraft zum Reissen gebracht werden kann, um den Kopfteil 2 vom Basisteil 1 zu trennen. Die Sollbruchstelle 4 kann in Gestalt einer umfänglich sich erstreckenden Querschnittsverengung oder einer Vielzahl umfänglich verteilt angeordneter, axial sich erstreckender Stege ausgebildet sein. Auch andere geeignete Ausbildungen der Sollbruchstelle 4 können vorgesehen sein.

Die im Basisteil 1 eingebettete Lanzettennadel 3 ragt einerends mit einem zugespitzten Endbereich 5 aus dem Basisteil 1 heraus. Die Lanzettennadel 3 besteht vorzugsweise aus einem geeigneten metallischen Material wie einem Stahlmaterial. Der Endbereich 5 ist bei intakter Sollbruchstelle 4 im Kopfteil 2 hermetisch gegenüber der Aussenumgebung abgeschirmt. Während die Lanzettennadel 3 mit Festsitz im Basisteil 1 gehalten ist, kann der Endbereich 5 der Lanzettennadel 3 mit weniger festen Sitz im Kopfteil 2 einsitzen, so dass der Kopfteil 2 nach der Entfernung vom Basisteil 1 vom Endbereich 5 der Lanzettennadel 3 getrennt werden kann, ohne dass bei diesem Vorgang eine wesentliche Kraft auf die Lanzettennadel 3 ausgeübt werden braucht. Im Rahmen der Erfindung ist jedoch auch ein Festsitz des Endbereiches 5 der Lanzettennadel 3 im Kopfteil 2 eingeschlossen.

Von einer Stirnfläche 6 des Kopfteils 2 steht axial in Richtung auf den Basisteil 1 ein Paar Nockenbereiche 7 ab, und von einer gegenüberliegenden Stirnfläche 8 des Basisteils 1 steht ein paar Nockenbereiche 9 axial in Richtung auf den Kopfteil 2 ab. Die Nockenbereiche 7 und 9 jedes Paares sind vorzugsweise diametral zueinander angeordnet, wobei die Nockenbereiche 7 eines Paares in die Lücken zwischen den Nockenbereichen 9 des anderen Paares eingreifen, wenn auf die Lanzette keine äussere Kraft einwirkt.

Jeder Nockenbereich 7, 9 kann eine spitzgiebelartige Konfiguration mit beidseitig eines Scheitelpunktes schräg gegen die betreffende Stirnfläche 6, 8 des Kopfteiles 2 bzw. Basisteiles 1 verlaufenden Nockenführungsflächen 11, 11' bzw. 12, 12' haben. Zugewandte Nockenführungsflächen 11, 12 benachbarter Nockenbereiche 7, 9 können bei einer Relativverdrehung des Kopfteils 2 gegenüber dem Basisteil 1 in Eingriff miteinander treten und eine gesteuerte axiale Bewegung des Kopfteils 2 bewirken, indem auf den Kopfteil 2 zusätzlich zu einer Bewegung in Umfangs- oder Drehrichtung ein Axialbewegung weg vom Basisteil 1 ausgelöst wird, wenn die betreffenden Nockenführungsflächen 11, 12 aufeinander abgleiten. Bei diesem Vorgang wird demzufolge der Kopfteil 2 axial vom Basisteil 1 wegbewegt, ohne dass auf den Kopfteil 2 eine äussere axiale Ziehkraft ausgeübt werden muss. Vielmehr bewirkt die Umsetzung der Drehkraft in eine Axialbewegung des Kopfteils 2, dass der Endbereich 5 der Lanzettennadel 3 ohne weiteres ganz oder wenigstens so weit freigelegt wird, dass eine geringe Kraft ausreicht, um den Kopfteil 2 endgültig aus dem Verbund mit dem Endbereich 5 der Lanzettennadel 3 herauszubringen.

Der Scheitelpunkt jedes Nockenbereiches 7, 9 ist ferner in einem bestimmten axialen Abstand von der betreffenden gegenüberliegenden Stirnfläche 6 bzw. 8 des Kopfteils 2 bzw. Basisteils 1 angeordnet. Hierdurch wird eine Anschlagbegrenzung bei einer seitlichen Verlagerung des Kopfteils 2 relativ zum Basisteil 1 bzw. aus einer axial ausgerichteten Beziehung zur Lanzettennadel 3 geschaffen. Eine auf den Kopfteil 2 absichtlich oder unabsichtlich ausgeübte seitliche Kraft kann daher nur zu einer begrenzten seitlichen Verlagerung des Kopfteils 2 aus der vorgegebenen axialen Ausrichtung führen. Diese Verlagerung kann durch eine ensprechende Abstandswahl zwischen den Scheitelpunkten der Nockenbereiche 7, 9 und der betreffenden Stirnfläche 6, 8 so bestimmt werden, dass auf die Lanzettennadel 3, insbesondere auf deren im Kopfteil 2 eingebetteten Endbereich 5 durch derartige seitliche Kräfte keine schädliche Auswirkungen hervorgerufen werden können.

Der übrige Aufbau der Lanzette ist dem Fachmann grundsätzlich bekannt, so dass sich eine Beschreibung diesbezüglicher Details erübrigt.

Die Erfindung wurde vorausgehend anhand einer Ausführungsform beschrieben, bei der die Nockenbereiche am Kopfteil und Basisteil identisch ausgebildet sind. Die Nockenbereiche am Kopfteil könnten jedoch auch unterschiedlich zu denen am Basisteil sein, indem z.B. die Nockenbereiche eines der Teile wie bei der vorbeschriebenen Ausführungsform ausgebildet sind und die Nockenbereiche am anderen Teil die Konfiguration von Stempeln oder Stutzen haben können, die axial von der betreffenden Stirnfläche abstehen und mit abgerundeter Endflächen versehen sein können. Ferner brauchen die Nockenführungsflächen der Nockenbereiche nicht axialbewegungsauslösend in beiden Drehrichtungen sein. Vielmehr könnte eine Trennung des Kopfteils vom Basisteil die Ausübung einer Drehkraft in einer bestimmten Drehrichtung voraussetzen. Vorausgehend wurde je ein Paar Nockenbereiche am Kopfteil als auch Basisteil beschrieben, die jeweils in einem Winkelabstand von 180° voneinander am betreffenden Teil vorgesehen sind. Stattdessen könnten auch mehr als zwei, z.B. drei Nockenbereiche in einem Winkelabstand von 60° an jedem Teil angeordnet sein. Schliesslich kann die Sollbruchstelle so ausgebildet sein, dass sie bei Ausübung einer Drehkraft auf den Kopfteil durchtrennt wird, bevor die Nockenbereiche in einen axialbewegungsauslösenden Eingriff treten, so dass durch die Nockenbereiche im Wesentlichen nur die Axialbewegung des Kopteils relative zum Basisteil gesteuert wird. Die Sollbruchstelle könnte jedoch auch so ausgelegt werden, dass das Durchtrennen erst im Zuge der Axialbewegung erfolgt oder in Verbindung damit fertiggestellt wird.

## Patentansprüche

1. Lanzette für die Blutentnahme, mit einer in einem Kunststoff-Basisteil (1) gehaltenen Lanzettennadel (3) aus einem metallischen Material, von der ein angespitzter Endbereich (5) aus dem Basisteil herausragt, wobei die Lanzettennadel in einem am Basisteil über eine Sollbruchstelle (4) angeformten Kunststoff-Kopfteil (2) eingebettet ist, der vor Gebrauch unter Freilegen des angespitzten Endbereiches der Lanzettennadel vom Basisteil entfernbar ist, wenn auf einen der Teile eine Drehkraft relative zum anderen Teil ausgeübt wird, **gekennzeichnet durch** zwischen dem Kopfteil (2) und dem Basisteil (1) angeordnete Nockenführungsmittel (7,9), die bei einer Relativdrehung zwischen dem Kopfteil und dem Basisteil miteinander in Eingriff bringbar sind, um eine axiale Bewegung des Kopfteiles relativ zum Basisteil zu bewirken.

2. Lanzette nach Anspruch 1, **dadurch gekennzeichnet, dass** die Nockenführungsmittel wenigstens ein Paar versetzt zueinander angeordnete Nockenbereiche (7) am Kopfteil (2) und wenigstens ein Paar versetzt zueinander und zu den Nockenbereichen am Kopftteil angeordnete Nockenbereiche (9) am Basisteil (1) umfassen, die mit den Nockenbereichen am Kopfteil bei der Drehung in Eingriff bringbar sind, wobei wenigstens die Nockenbereiche eines Paars Nockenführungsflächen (11, 11',12,12') aufweisen.

3. Lanzette nach Anspruch 1 oder 2, **dadurch gekennzeichnet, dass** die Nockenbereiche (7,9) in wenigstens einer Drehrichtung axialbewegungsauslösend wirksam sind.

4. Lanzette nach einem der vorhergehenden Ansprüche, **dadurch gekennzeichnet, dass** die vorstehenden Enden der Nockenbereiche (7,9) in einem solchen Abstand von einer zugewandten Stirnfläche (6,8) des jeweiligen gegenüberliegenden Teiles [Basisteil (1) oder Kopfteil (2)] angeordnet sind, dass eine seitliche Bewegung eines der Teile gegenüber dem anderen aus einer koaxialen Ausrichtung zur Lanzettennadel (3) begrenzt ist.

## Claims

1. A lancet for the extraction of blood, including a lancet needle (3) of metallic material held in a base part (1) of plastics material, a pointed end portion (5) of said lancet needle protruding from said base part, said lancet needle being embedded in a head part (2) of plastics material being formed to the base part by a breaking zone (4) and adapted to be removed from the base part thereby exposing the pointed end portion of the lancet needle by exerting a rotational force to one of said base and head parts relative to the other, **characterized by** cooperating cam means (7,9) disposed between said head part (2) and said base part (1), said cam means engaging with each other upon relative rotation between the head and base parts for causing an axial movement of the head part relative to the base part.

2. The lancet according to claim 1, **characterized in that** said cam means comprise at least a pair of cam portions (7) provided on said head part (2) and offset with each other, and at least a pair of cam portions (9) provided on said base part (1) and offset with each other and also offset relative to the cam portions of said head part, said cam portions of the head and base parts engaging with each other during said rotation, whereby at least the cam portions of one said pairs of cam portions having cam guiding faces (11,11',12,12').

3. The lancet according to claim 1 or 2, **characterized in that** said cam portions (7,9) are effective for initiating an axial movement in at least one direction of said rotation.

4. The lancet according to one of the preceding claims, **characterized in that** said cam portions (7,9) have top ends spaced from an adjacent one of a facing end face (6,8) of the associated opposite part [base part (1) or head part (2)] in such manner that a lateral movement of one of said parts relative to the other from a coaxial alignment relative to the lancet needle (3) is limited.

## Revendications

1. Lancette de prélèvement de sang, comportant une aiguille de lancette (3) maintenue dans un élément de base en matière plastique (1) et fabriquée dans un matériau métallique, dont une zone d'extrémité pointue (5) dépasse de l'élément de base, l'aiguille de lancette étant encastrée dans un élément de tête ou formant tête (2) en matière plastique formé sur l'élément de base par l'intermédiaire d'un point de rupture (4), qui, avant utilisation, peut être séparé de l'élément de base en dégageant la zone d'extrémité pointue de l'aiguille de lancette lorsqu'une force de rotation est exercée sur l'un des deux éléments par rapport à l'autre, **caractérisée par** des moyens de guidage d'ergots (7, 9) disposés entre l'élément de tête (2) et l'élément de base (1 ), qui peuvent être amenés à s'engrener réciproquement lors d'un mouvement de rotation relatif entre l'élément de tête et l'élément de base, de façon à générer un mouvement axial de l'élément de tête par rapport à l'élément de base.

2. Lancette selon la revendication 1, **caractérisée en ce que** les moyens de guidage des ergots comportent, au niveau de l'élément de tête (2), au moins une paire de zones d'ergots (7) décalées entre elles et, au niveau de l'élément de base (1), au moins une paire de zones d'ergots (9) décalées entre elles et par rapport aux zones d'ergots de l'élément de tête, qui lors d'un mouvement de rotation peuvent être amenées à s'engrener avec les zones d'ergots de l'élément de tête, les zones d'ergots d'une paire, au moins, présentant des surfaces de guidage d'ergots (11, 11' ; 12, 12').

3. Lancette selon la revendication 1 ou 2, **caractérisée en ce que** les zones d'ergots (7, 9) sont efficaces pour provoquer un mouvement axial, dans un sens de rotation au moins.

4. Lancette selon l'une des revendications précédentes, **caractérisée en ce que** les extrémités faisant saillie des zones d'ergots (7, 9) présentent un écart tel par rapport à une face frontale associée (6, 8) de la partie respectivement opposée [élément de base (1) ou élément de tête (2)], qu'un mouvement latéral, selon une direction coaxiale à l'aiguille de lancette (3), de l'un des éléments par rapport à l'autre est limité.
